# EUROPEAN PATENT APPLICATION

(11) **EP 4 684 812 A1**
(43) Date of publication of application: **28.01.2026**
(21) Application number: 24775196.9
(22) Date of filing: 20.03.2024
(51) Int. Cl.: A61L 27/16, A61L 27/52, C09D 11/101, C09D 11/03, B33Y 70/00, B33Y 80/00

(54) **3D PRINTING INK COMPOSITION AND 3D PRINTING MOLDED ARTICLE FORMED BY CURING SAME**

(30) Priority: 21.03.2023 KR 20230036488
(71) Applicant: Aldaver Inc., Daejeon 34051 (KR)
(72) Inventor: LEE, Taehoon, Daejeon 34052 (KR); KIM, Jin-Oh, Daejeon 34040 (KR); KIM, Ikjin, Daejeon 34052 (KR); LEE, Ye Rim, Daejeon 35202 (KR); KO, Kun Woo, Yongin-si, Gyeonggi-do 16939 (KR)
(74) Representative: dompatent
(86) International application number: PCT/KR2024/003489
(87) International publication number: WO 2024/196149

(57) **Abstract**

The present invention relates to a 3D printing ink composition including a pluronic copolymer, an acrylamide monomer, an alkali metal salt and a solvent, and a 3D printing molded article formed by curing the ink composition.

## Description

### [Technical Field]

The present application claims the benefits of priority to Korean Patent Application No. 10-2023-0036488 filed on March 21, 2023, the entire contents of which are incorporated as part of this specification.

The present invention relates to a 3D printing ink composition and a 3D printing molded article formed by curing the same.

### [Background Art]

A 3D (3-dimension) printer is a device that produces an actual 3-dimensional shape intactly based on a 3-dimensional drawing input as if to print letters or pictures. 3D printing technology has recently become a very hot issue and expanded into the fields of automobiles, medicine, art and education, and is widely used for the purpose of creating various models. Principles of 3D printers may be largely divided into a cutting type and a stacking type, and most of actually used 3D printers correspond to a stacking type having no material loss.

There are about 20 methods that use a stacking-type principle, however, the most commonly used methods are SLA (stereo lithography apparatus), FDM (fused deposition modeling), FFF (fused filament fabrication) and SLS (selective laser sintering) methods.

SLA is a method of modeling by projecting a laser beam into a water tank holding a liquid-state photocurable resin, and epoxy-type photopolymers, which are photocurable resins, are mainly used. On the other hand, FDM (or FFF), a method in which an introduced filament-type material is 3-dimensionally modeled while being discharged in a molten state from a nozzle of a printer moving along X, Y and Z axis, uses thermoplastic plastic as a main material. Meanwhile, SLS implements 3D printing by selective sintering through shooting a laser to a water tank holding a powder-type material such as metal, plastic and ceramic powder.

Among the three methods, the FDM method preparing and using thermoplastic plastic in a filament form is most widely popularized since the price of 3D printer is relatively low and the printing speed is faster than that of other methods. In the FDM, rigid materials such as polylactic acid (PLA), ABS (acrylonitrile butadiene styrene), HDPE and polycarbonate (PC), and flexible materials such as thermoplastic elastomers are generally used due to reasons such as having excellent bed adhesive strength and interlayer adhesive strength when forming a 3D structure, and having favorable shape stability.

Recently, development of so called a '3D bioprinting technology', which is a technology forming artificial tissues or organs through materials such as hydrogels using a DIW (direct ink writing) method of extruding and stacking an ink in a manner similar to the FDM method, is also actively ongoing.

However, hydrogels have problems in that, since the material itself includes a large amount of moisture, properties of a molded article after printing are weak to support external force, and it is not easy to use various types of materials at the same time in the processing process.

Accordingly, there is a need to develop an ink composition capable of securing mechanical properties of a 3D molded article while allowing the use of the DIT-type discharge-based printing technology.

### [Disclosure]

### [Technical Problem]

It is an object of the present invention to provide a 3D printing ink composition that is an ink composition that may be used for 3D printing, which includes a hydrogel that is a biocompatible polymer, and a certain amount of an alkali metal salt, so that a molded article formed by curing the composition may secure mechanical properties sufficient to support external force.

It is another object of the present invention to provide a 3D printing molded article formed by curing the composition and having improved elastic modulus (Young's modulus) and ultimate tensile strength.

### [Technical Solution]

One embodiment of the present invention provides a 3D printing ink composition including: a pluronic copolymer; an acrylamide monomer; an alkali metal salt; and a solvent.

The pluronic copolymer may be represented by Formula 1 below. 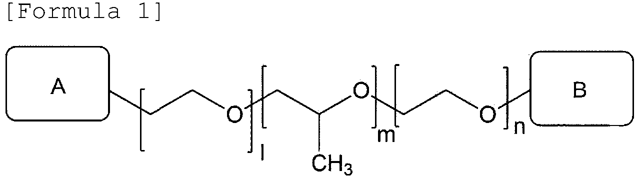 wherein,
A and B are terminal groups capable of forming a bond with the acrylamide monomer, and
l is an integer of 95 to 100, m is an integer of 62 to 69, and n is an integer of 95 to 100.

A of Formula 1 may be represented by Structural Formula A-1 or Structural Formula A-2, and B of Formula 1 below may be represented by Structural Formula B-1 or Structural Formula B-2 below.

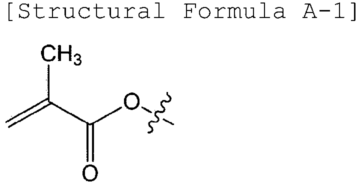

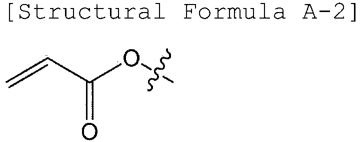

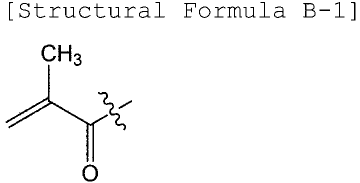

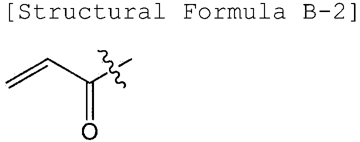

In Structural Formula A-1 to Structural Formula B-2, 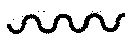 represents a site linked to Formula 1.

The acrylamide monomer may be at least one selected from the group consisting of acrylamide, N-isopropylacrylamide (NIPAM), N-tert-butylacrylamide, N,N-diethylacrylamide, N-ethylacrylamide, N-n-propylacrylamide, N,N-ethylmethylacrylamide, N-isopropylmethacrylamide, N-hydroxyethylacrylamide, N-(isobutoxymethyl)acrylamide, N-tert-butyl methacrylamide, N,N-diethyl methacrylamide and N-ethyl methacrylamide.

The alkali metal salt may include a combination of at least one anion selected from the group consisting of OH⁻, SO₄²⁻, CO₃²⁻, BrO₃⁻, Cl⁻, OAc⁻, IO₃⁻, Br⁻, I⁻ and NO₃⁻, and at least one cation selected from the group consisting of Na⁺, K⁺, Li⁺, Ba²⁺, Rb⁺, Ca²⁺, Ni²⁺, Co²⁺, Mg²⁺, Fe²⁺, Zn²⁺, Cs⁺, Mn²⁺, Al³⁺, NH⁴⁺ and H⁺.

The composition may further include a crosslinking agent and a photoinitiator.

The crosslinking agent may be at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin and ethylene carbonate.

The photoinitiator may be at least one selected from the group consisting of IRGACURE 2959 (2-hydroxy-1-[4-(2-hydroxyethoxy)phenyl]-2-methyl-1-propanone), IRGACURE 500 (1-hydroxy-cyclohexyl-phenyl-ketone and benzophenone) and IRGACURE 754 (oxy-phenyl-acetic acid 2-[2-oxo-2-phenyl-acetoxy-ethoxy]-ethyl ester and oxy-phenyl-acetic 2-[2-hydroxy-ethoxy]-ethyl ester).

The pluronic copolymer may be included in an amount of 15 parts by weight to 80 parts by weight based on 100 parts by weight of the solvent.

The acrylamide monomer may be included in an amount of 5 parts by weight to 100 parts by weight based on 100 parts by weight of the solvent.

The alkali metal salt may be included in an amount of greater than 0 parts by weight and 25 parts by weight or less based on 100 parts by weight of the solvent.

The crosslinking agent may be included in an amount of 0.0037 parts by weight to 0.074 parts by weight based on 100 parts by weight of the solvent.

The photoinitiator may be included in an amount of 0.04 parts by weight to 0.4 parts by weight based on 100 parts by weight of the solvent.

The acrylamide monomer and the alkali metal salt in the composition may have a weight ratio of 2:1 to 20:1.

The composition may have a gelation temperature of 5°C to 60°C.

Another embodiment of the present invention provides a 3D printing molded article formed by curing the composition.

The 3D printing molded article may have elastic modulus of 0.15 MPa to 1.3 MPa.

The 3D printing molded article may have ultimate tensile strength of 0.05 MPa to 0.8 MPa.

### [Advantageous Effects]

An ink composition that can be used for 3D printing according to the present invention includes a hydrogel, which is a biocompatible polymer, together with a pluronic copolymer, and further includes a certain amount of an alkali metal salt, so that rheological properties sufficient to support external force as a printing structure before curing the composition is provided, and a material disadvantage of the hydrogel having properties not sufficient to support external force by including a large amount of moisture despite an advantage of providing an environment advantageous for cell proliferation and differentiation even after curing the composition is supplemented. Accordingly, sufficient mechanical properties, particularly, improved elastic modulus and ultimate tensile strength, of a molded article can be secured.

### [Description of Drawings]

FIGS. 1 and 2 each illustrate one embodiment of a 3D printing molded article formed through a 3D printing ink composition according to one embodiment of the present disclosure.
FIG. 3 is a schematic diagram illustrating a process in which a pluronic copolymer and an acrylamide monomer form a network to enable 3D printing through a 3D printing ink composition according to one embodiment of the present disclosure.
FIG. 4 is a schematic diagram illustrating a process in which a pluronic copolymer gelates while forming micelles in an aqueous solvent in a 3D printing ink composition according to one embodiment of the present disclosure.

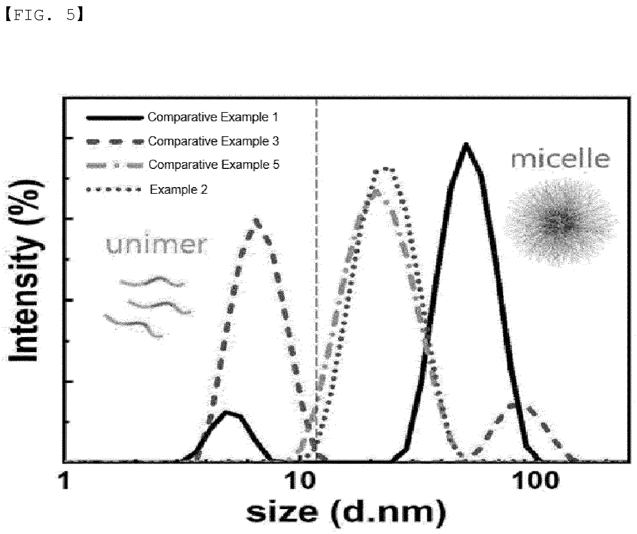

FIG. 5 is a graph illustrating a change in the average particle size of micelles depending on changes in the amounts of a pluronic copolymer, an acrylamide monomer and an alkali metal salt in each of compositions according to the example and the comparative example of the present disclosure.
FIG. 6 is a graph illustrating a change in the average particle size of micelles depending on a change in the amount of an acrylamide monomer in each of compositions according to example and comparative example of the present disclosure.
FIG. 7 is a graph illustrating a change in the average particle size of micelles depending on a change in the amount of an alkali metal salt in each of compositions according to example and comparative example of the present disclosure.
FIG. 8 is a graph illustrating a change in the average particle size of micelles depending on a change in the amount of an alkali metal salt in the presence of an acrylamide monomer in each of compositions according to example and comparative example of the present disclosure.
FIG. 9 is a graph illustrating a gelation temperature measured through storage modulus (G') and loss modulus (G") depending on a change in the temperature of each of compositions according to example and comparative example of the present disclosure.
FIG. 10 is a graph illustrating, in each of compositions according to example and comparative example of the present disclosure, a change in the gelation temperature of the composition depending on a change in the amount of an alkali metal salt in the presence of an acrylamide monomer.
FIG. 11 illustrates elastic modulus and ultimate tensile strength of a molded article formed by curing each of compositions according to example and comparative example of the present disclosure.
FIG. 12 illustrates one embodiment of a 3D printing molded article formed through a 3D printing ink composition according to one embodiment of the present disclosure.
FIG. 13 illustrates a result of visually identifying ultimate tensile strength of a 3D printing molded article formed through a 3D printing ink composition according to one embodiment of the present disclosure.
FIG. 14 is a graph illustrating a change in viscosity depending on a shear rate of each of 3D printing ink compositions according to example and comparative example of the present disclosure.
FIG. 15 is a graph illustrating a shear-thinning phenomenon of each of 3D printing ink compositions according to example and comparative example of the present disclosure.
FIG. 16 is a graph illustrating 3D printing controllability of each of 3D printing ink compositions according to example and comparative example of the present disclosure.
FIG. 17 illustrates images presenting a surgical simulation process of a molded article formed from each of 3D printing ink compositions according to example and comparative example of the present disclosure.
FIG. 18 illustrates fluorescence images that monitor cell death, which indicates biocompatibility of a 3D printing ink composition according to one embodiment of the present disclosure.
FIG. 19 is a graph illustrating biocompatibility of a 3D printing ink composition according to one embodiment of the present disclosure.
FIG. 20 is a graph comparing tensile strength of a molded article formed from each of 3D printing ink compositions according to example and comparative example of the present disclosure, with tensile strength of human blood vessel depending on the age group.

### [Best Mode]

Hereinafter, embodiments of the present invention will be described in detail. Before this, terms or words used in the present specification and the claims are not to be interpreted limitedly to common or dictionary meanings, and need to be interpreted as meanings and concepts corresponding to technical ideas of the present invention based on a principle in which the inventors may suitably define concepts of the terms in order to describe the invention in the best possible way. Accordingly, constitutions described in embodiments described in the present specification are just most preferred one embodiment of the present invention and do not represent all of the technical ideas of the present disclosure, and therefore, it needs to be understood that there may be various equivalents and modified examples that may replace these at the time of filing the present application.

Throughout the present specification, a description of a certain part "including" certain constituents means that it may further include other constituents, and does not exclude other constituents unless particularly stated on the contrary.

In addition, description of specifying constituents by limiting or adding constituents may be applied to all inventions unless there is a particular restriction, and is not limited to a specific invention.

In addition, throughout the description of invention and the claims of the present application, singular forms include plural forms as well unless otherwise stated.

In addition, throughout the description of invention and the claims of the present application, "or" includes "and" unless otherwise stated. Therefore, "including A or B" means all three cases of including A, including B, or including A and B.

In addition, all numerical ranges include values of both ends and all intermediate values therebetween, unless explicitly stated to exclude them.

Hereinafter, a 3D printing ink composition according to one embodiment of the present invention will be described.

### 3D Printing Ink Composition

The present invention relates to a 3D printing ink composition including a hydrogel, which is a biocompatible polymer, together with a pluronic copolymer, and further including a certain amount of an alkali metal salt, so that rheological properties sufficient to support external force as a printing structure before curing the composition, particularly, properties of readily gelling the composition at room temperature without introducing a separate heat source, may be provided, and a material disadvantage of the hydrogel having properties not sufficient to support external force by including a large amount of moisture despite an advantage of providing an environment advantageous for cell proliferation and differentiation even after curing the composition is supplemented, and sufficient mechanical properties, particularly, improved elastic modulus and ultimate tensile strength, of a molded article may be secured.

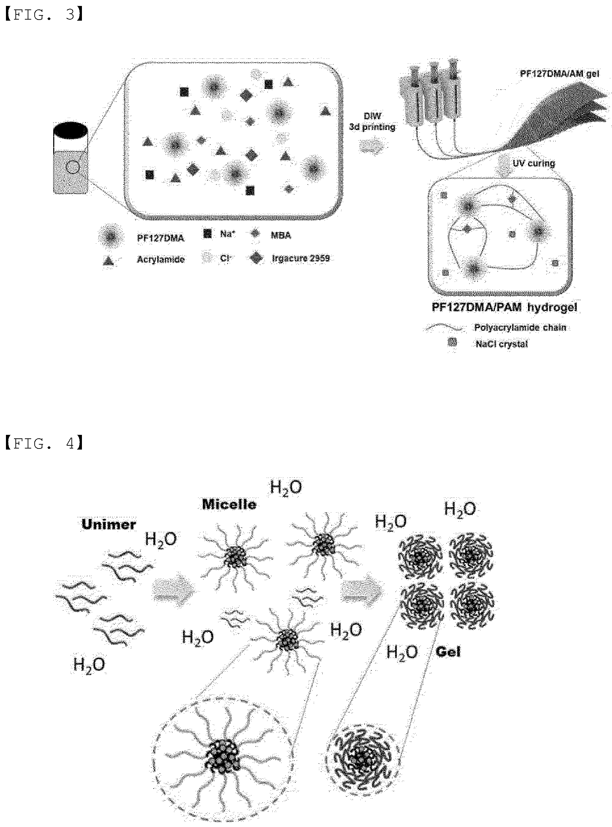

FIG. 3 is a schematic diagram illustrating that each composition included in the 3D printing ink composition according to one embodiment of the present invention may be used in 3D printing by forming a network through a UV curing process.

Referring to FIG. 3, the 3D printing ink composition according to one embodiment of the present invention includes a pluronic copolymer, an acrylamide monomer, an alkali metal salt and a solvent.

Hereinafter, each component of the 3D printing ink composition according to one embodiment of the present invention will be described.

### (1) Pluronic Copolymer

As the pluronic copolymer, various copolymers sold under the trade name of Pluronic by BASF Corporation may be used, and examples thereof may include various copolymers such as F127 and P105, and derivatives thereof, however, the pluronic copolymer is not limited thereto. For example, the pluronic copolymer may be Pluronic F127 represented by Formula A below or a derivative thereof.

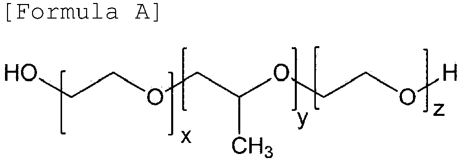

Pluronic F127 or a derivative thereof is a biocompatible synthetic polymer and an amphiphilic material having both a hydrophilic group and a hydrophobic group, and may have various forms depending on a solvent or a concentration state when mixed. Such properties of Pluronic F127 allow sol-gel transition in an underwater state, making it possible to apply to 3D bioprinting with the addition of biocompatible properties.

In one embodiment of the present disclosure, the pluronic copolymer may be represented by Formula 1 below. 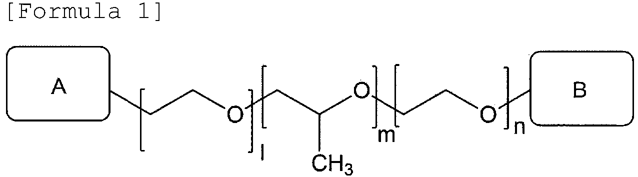 wherein,
A and B are terminal groups capable of forming a bond with the acrylamide monomer,
l is an integer of 95 to 100, m is an integer of 62 to 69, and n is an integer of 95 to 100.

In this case, A of Formula 1 is represented by Structural Formula A-1 or Structural Formula A-2 below, and
B of Formula 1 may be represented by Structural Formula B-1 or Structural Formula B-2 below.

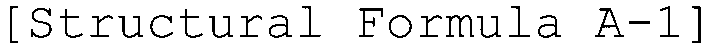

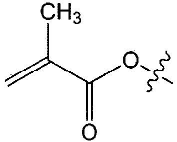

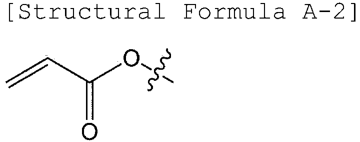

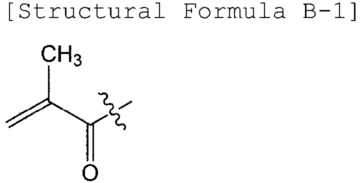

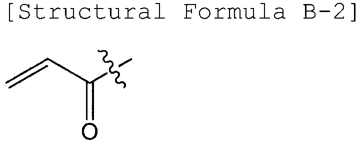

In Structural Formula A-1 to Structural Formula B-2, 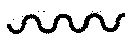 represents a site linked to Formula 1.

In other words, when the pluronic copolymer is the polymer represented by Formula 1, it may be a di (meth)acrylated pluronic copolymer further including a substituent capable of bonding to the acrylamide monomer, which is another molecule in the composition having a substituent capable of intermodular bonding, at both ends of Pluronic F127 having excellent printability of 3D printing.

In addition, the pluronic copolymer also has properties of performing a role of a crosslinking agent to be described later by including a substituent capable of intramolecular bonding at both ends.

Specifically, the pluronic copolymer represented by Formula 1 may be prepared through Reaction Formula 1-1 or Reaction Formula 1-2 below according to previous reports such as W. I. Choi, G. Tae, Y. H. Kim, One pot, single phase synthesis of thermo-sensitive nano-carriers by photo-crosslinking of a diacrylated pluronic, J. Mater. Chem., 2008, 18, 2769-2774. 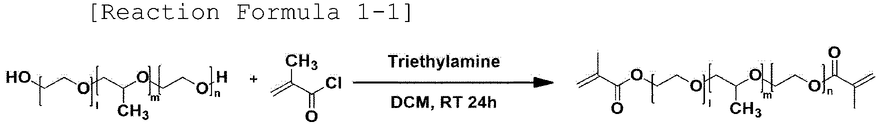 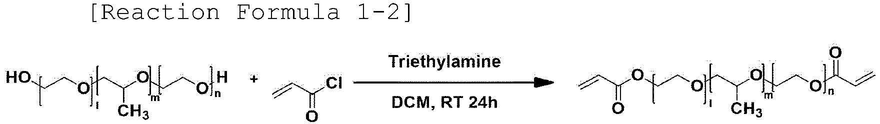 (In Reaction Formulae 1-1 and 1-2, l is an integer of 95 to 100, m is an integer of 62 to 69, and n is an integer of 95 to 100.)

In other words, Pluronic F127, triethylamine and (meth)acryloyl chloride are introduced to an organic solvent such as dichloromethane (DCM) or anhydrous toluene, and the mixture may be stirred to prepare the pluronic copolymer represented by Formula 1.

In the reaction, Pluronic F127 may be introduced in a ratio of 50 g to 150 g or 75 g to 125 g, and preferably 90 g to 110 g based on 1,000 mL of the solvent. When the content of Pluronic F127 is less than the above-mentioned range, the amount of the solvent is excessively large, and when the content of Pluronic F127 is greater than the above-mentioned range, Pluronic F127 may not be completely dissolved in the solvent.

In the reaction, the triethylamine may be introduced in a ratio of 3 mL to 12 mL or 4 mL to 10 mL, and preferably 3 mL to 12 mL based on 1,000 mL of the solvent. When the content of the triethylamine is less than the above-mentioned range, deprotonation of Pluronic F127 may not completely proceed, causing a problem of not being able to fully introduce the (meth)acrylate group to both ends of Pluronic F127, and when the content of the triethylamine is greater than above-mentioned content range, the deprotonation may proceed excessively, requiring an additional washing procedure.

In the reaction, the (meth)acryloyl chloride may be introduced in a ratio of 2.5 mL to 7 mL or 3 mL to 6 mL, and preferably 3.5 mL to 5 mL based on 1,000 mL of the solvent. When the content of the (meth)acryloyl chloride is less than the above-mentioned range, the (meth)acrylate group may not be fully introduced to both ends of Pluronic F127, and when the content of the (meth)acryloyl chloride is greater than the above-mentioned content range, unnecessary residue may exist even after preparing the pluronic copolymer represented by Formula 1, and an additional washing procedure may be required.

In the process of preparing the pluronic copolymer represented by Formula 1 through Reaction Formula 1-1 or 1-2, a process of washing the product using an ether-based solvent may be further included. In this case, the washing process may be performed while sufficiently stirring the product two or more times using an ether-based solvent in an amount of 500 mL or more.

In one embodiment of the present disclosure, the pluronic copolymer may be included in an amount of 15 parts by weight to 80 parts by weight based on 100 parts by weight of the solvent included in the composition, and the amount may be, for example, 15 parts by weight or more, 20 parts by weight or more, 25 parts by weight or more, 30 parts by weight or more, 35 parts by weight or more or 40 parts by weight or more, and 80 parts by weight or less, 75 parts by weight or less, 70 parts by weight or less, 65 parts by weight or less, 60 parts by weight or less or 55 parts by weight or less. When the content of the pluronic copolymer is less than 15 parts by weight based on 100 parts by weight of the solvent, effective 3D printing may not be achieved since gelation of the composition is inhibited, and when the content is greater than 80 parts by weight, the pluronic copolymer may not be smoothly dissolved in the solvent.

### (2) Acrylamide Monomer

The 3D printing ink composition according to the present invention further includes an acrylamide monomer together with the pluronic copolymer. The acrylamide monomer is one type of hydrogel, and changes into polyacrylamide after the composition according to the present invention is cured. The acrylamide monomer bonds to A or B of the pluronic copolymer represented by Formula 1 according to one embodiment of the present invention to form a hydrogel network, and performs a role of supplementing a disadvantage of the pluronic copolymer, the advantage being readily dissolved in water without being cured.

Specifically, the pluronic copolymer represented by Formula 1 has a structure of 'hydrophilic region 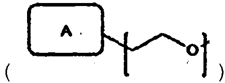 '-'hydrophobic region 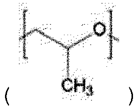 '-'hydrophilic region 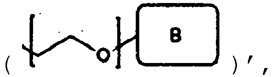 and forms a so-called "micelle" structure in which the hydrophilic region is disposed outward and the hydrophobic region is disposed inward in an aqueous solvent. In this case, the acrylamide monomer forms a network by bonding to the hydrophilic region of the pluronic copolymer represented by Formula 1 forming a micelle structure through radical polymerization and the like, and after forming the network, a hydrogen bond is formed between the polyacrylamide and the micelle structure of the pluronic copolymer, increasing mechanical properties of a molded article of the 3D printing ink composition.

In one embodiment of the present disclosure, the acrylamide monomer may be at least one selected from the group consisting of acrylamide, N-isopropylacrylamide (NIPAM), N-tert-butylacrylamide, N,N-diethylacrylamide, N-ethylacrylamide, N-n-propylacrylamide, N,N-ethylmethylacrylamide, N-isopropylmethacrylamide, N-hydroxyethylacrylamide, N-(isobutoxymethyl)acrylamide, N-tert-butyl methacrylamide, N,N-diethyl methacrylamide and N-ethyl methacrylamide, and for example, acrylamide may be used.

In one embodiment of the present disclosure, the acrylamide monomer may be included in an amount of 5 parts by weight to 100 parts by weight based on 100 parts by weight of the solvent included in the composition, and the amount may be, for example, 5 parts by weight or more, 10 parts by weight or more, 15 parts by weight or more, 20 parts by weight or more, 25 parts by weight or more, 30 parts by weight or more, 35 parts by weight or more, 38.46 parts by weight or 40 parts by weight or more, and 100 parts by weight or less, 95 parts by weight or less, 90 parts by weight or less, 85 parts by weight or less, 80 parts by weight or less, 75 parts by weight or less, 70 parts by weight or less, 65.94 parts by weight or less, 65 parts by weight or less, 60 parts by weight or less or 55 parts by weight or less. When the content of the pluronic copolymer is less than 5 parts by weight based on 100 parts by weight of the solvent, the acrylamide monomer may not be sufficiently cured, and properties such as elastic modulus of a molded article formed by curing the composition may not meet the desired range, and when the content is greater than 100 parts by weight, the sol nature of the composition becomes excessively high, causing a problem of not being suitable for 3D printing.

### (3) Alkali Metal Salt

The 3D printing ink composition according to the present invention further includes an alkali metal salt together with the pluronic copolymer and the acrylamide monomer.

The 3D printing ink composition according to the present invention may be effectively used in extrusion-type 3D printing methods, and has an advantage of being more suitable for DIW (direct ink writing)-type printing among these when a composition with a relatively soft material is used.

Even when the DIW-type printing is used, a 3D printing molded article may not be stably formed when the 3D printing ink composition is in a sol state since the composition may flow from, for example, a nozzle unit of the 3D printing device, and therefore, it is important for the 3D printing ink composition to maintain a gel state.

The pluronic copolymer of the 3D printing ink composition according to the present invention may undergo a sol-gel transition (gelation) depending on the temperature and the unimer concentration.

Referring to FIG. 4, the pluronic copolymer in a unimer state has the hydrophilic region 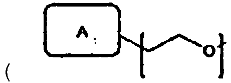 and 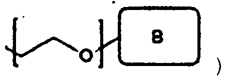 of the copolymer disposed outward and the hydrophobic region 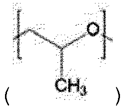 of the copolymer disposed inward in an aqueous solvent environment, forming a micelle structure, and properties of a 'gel' state are further enhanced when the micelle structure of the pluronic copolymer is formed in large quantities.

However, the 3D printing ink composition according to the present invention further includes an acrylamide monomer in order to supplement the disadvantage of the pluronic copolymer being readily dissolved in water without being cured and to secure mechanical properties of a 3D printing molded article, and since the acrylamide monomer has 'sol' properties, 'gel' properties of the composition including the same tend to be weakened. Accordingly, when the composition includes the acrylamide monomer together with the pluronic copolymer, micelle formation of the pluronic copolymer is inhibited in an aqueous solvent environment, causing a property of returning the copolymer to its unimer state.

The alkali metal salt of the 3D printing ink composition according to the present invention is included to enhance 'gel' properties suitable for 3D printing by weakening 'sol' properties resulting from the composition further including the acrylamide monomer as described above.

Although not limited to a specific theory, such a phenomenon may be understood such that, by the 3D printing ink composition further including the alkali composition, activity of the aqueous solvent is reduced, inducing the effect of reducing solubility for each composition in the composition, and as a result, tendency of the pluronic copolymer to form micelles increases, and 'gelation' of the entire composition is induced.

Accordingly, whereas a separate heat source needs to be introduced in order to 'gelate' a material in a 'sol' state in general, the 3D printing ink composition according to the present invention has a reduced gelation temperature for the entire composition by further including a certain amount of the alkali metal salt, thereby exhibiting 'gel' properties even at room temperature. Therefore, the composition may be suitable for 3D printing, for example, DIW-type printing.

When the gelation temperature of the composition is higher than room temperature, a separate heat source needs to be supplied to the composition in order to proceed with printing using a 3D printing device, and it may be difficult to provide stable properties to a 3D printing structure as the composition exhibits a 'sol' tendency in general since, for example, heat is not sufficiently transferred to a nozzle unit of the 3D printing device.

In one embodiment of the present disclosure, the alkali metal salt may include a combination of at least one anion selected from the group consisting of OH⁻, SO₄²⁻, CO₃²⁻, BrO₃⁻, Cl⁻, OAc⁻, IO₃⁻, Br⁻, I⁻ and NO₃⁻ and at least one cation selected from the group consisting of Na⁺, K⁺, Li⁺, Ba²⁺, Rb⁺, Ca²⁺, Ni²⁺, Co²⁺, Mg²⁺, Fe²⁺, Zn²⁺, Cs⁺, Mn²⁺, Al³⁺, NH⁴⁺ and H⁺, and for example, the alkali metal salt may be NaCl.

In addition, in one embodiment of the present disclosure, the alkali metal salt may be included in an amount of greater than 0 parts by weight and 25 parts by weight or less based on 100 parts by weight of the solvent included in the composition, and the amount may be, for example, 1 part by weight or more, 2 parts by weight or more, 3 parts by weight or more, 4 parts by weight or more, 5 parts by weight or more, 5.81 parts by weight or more, 6 parts by weight or more, 7 parts by weight or more, 8 parts by weight or more, 9 parts by weight or more or 10 parts by weight or more, and 25 parts by weight or less, 24 parts by weight or less, 23 parts by weight or less, 22 parts by weight or less, 21 parts by weight or less, 20.35 parts by weight or less, 20 parts by weight or less, 19 parts by weight or less, 18 parts by weight or less, 17 parts by weight or less, 16 parts by weight or less or 15 parts by weight or less.

When the content of the alkali metal salt satisfies the above-mentioned range, the composition exists in a gel state at room temperature, which may be suitable for a 3D printing environment.

In one embodiment of the present disclosure, the acrylamide monomer and the alkali metal salt in the composition may have a weight ratio 2:1 to 20:1, and the weight ratio may be, for example, 2.25:1 to 15:1, 2.5:1 to 12:1, 3:1 to 10:1, and preferably 3.25:1 to 8:1. When the weight ratio of the acrylamide monomer with respect to the alkali metal salt is greater than 20:1 in the composition, the gelation temperature is higher than room temperature, resulting in a strong 'sol' tendency at room temperature, and there may be problems in that the composition may flow down, failing to maintain the shape during printing, and stability of a 3D structure may not be secured until the curing process is performed after printing. When the weight ratio of the acrylamide monomer with respect to the alkali metal salt is less than 2:1 in the composition, the salt concentration significantly increases in the composition, and there may be problems in that the alkali metal salt is not favorably dissolved and not uniformly mixed as well, and it may be difficult to optimize printing variable control, bubble control and the like as the 'gel' tendency excessively increases.

In one embodiment of the present disclosure, the 3D printing ink composition may have a gelation temperature of 5°C to 60°C, and the gelation temperature may be, for example, 5°C or higher, 10°C or higher or 15°C or higher, and 60°C or lower, 55°C or lower, 50°C or lower, 45°C or lower, 40°C or lower, 35°C or lower, 30°C or lower or 25°C or lower. By the 3D printing ink composition according to the present invention further including the alkali metal salt together with the pluronic copolymer and the acrylamide monomer, the entire composition has a reduced gelation temperature, and an advantage of performing stable 3D printing even at room temperature in the above-mentioned range is obtained.

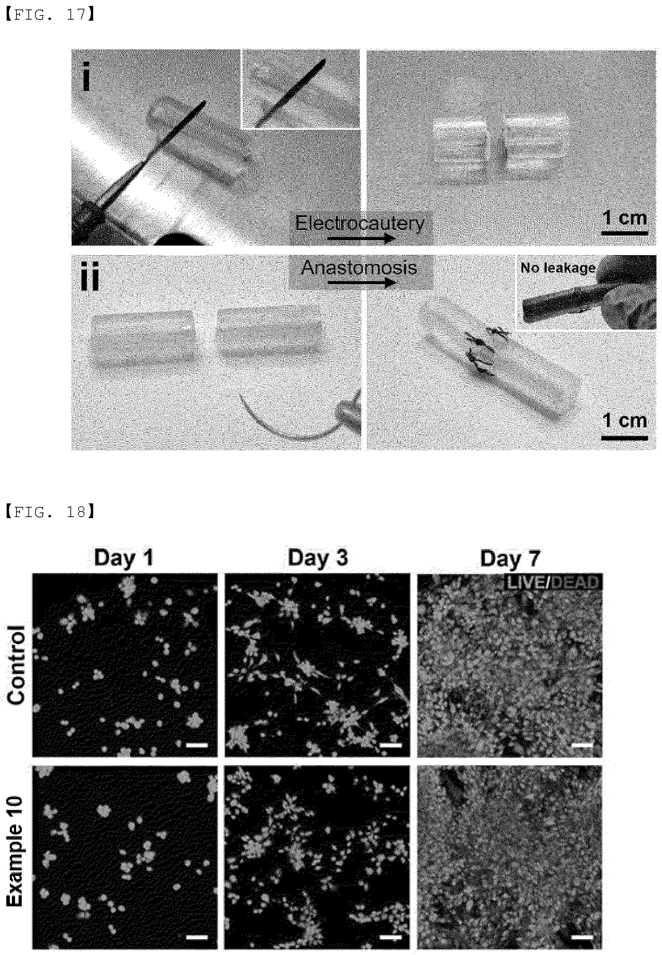

In addition thereto, by the 3D printing ink composition according to one embodiment of the present invention including the alkali metal salt, an effect of improving electric conductivity of a 3D printing molded article formed from the composition may also be provided. Accordingly, there is an advantage of more effectively using the composition in devices using electric energy among various molded articles that may be formed from the 3D printing ink composition. For example, as shown in FIG. 17, an artificial vessel formed from the 3D printing ink composition according to one embodiment of the present invention may be smoothly applied to a medical device using an electric device such as electrocautery as a result of improving electric conductivity with the introduction of the alkali metal salt into the composition. The mechanical properties are improved as well, and, during the process of surgical simulation such as anastomosis based on the artificial vessel, improved properties may be exhibited such that the artificial vessel does not tear or open, and the anastomosed area does not leak.

### (4) Other Components

The 3D printing ink composition of the present invention may further include a crosslinking agent and a photoinitiator in addition to the above-described components for property control.

The crosslinking agent is a material that acts as a crosslinker between the network formed through the pluronic copolymer and the acrylamide monomer. Specifically, the crosslinking agent crosslinks between the polyacrylamide chains formed by curing the acrylamide monomer, thereby performing a role of further improving mechanical strength and chemical stability of a 3D printing molded article.

Accordingly, the crosslinking agent is not limited in the type as long as it a material capable of crosslinking the network form through the pluronic copolymer and the acrylamide monomer of the present disclosure, and for example, at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin and ethylene carbonate may be used.

The crosslinking agent may be included in an amount of 0.0037 parts by weight to 0.074 parts by weight based on 100 parts by weight of the solvent of the composition according to the present disclosure, and for example, may be included in an amount of 0.0055 parts by weight to 0.062 parts by weight, and preferably 0.01 parts by weight to 0.05 parts by weight. When the content of the crosslinking agent is less than the above-mentioned range, sufficient crosslinking to provide mechanical strength and chemical stability to a 3D printing molded article may not be achieved, and when the content is greater than the above-mentioned range, the crosslinking is formed excessively, causing a concern of decline in the properties such as tensile.

The photoinitiator is a material used to initiate a chain reaction for curing the composition, and specifically, means a material that readily produces radicals using a light source such as UV.

The photoinitiator is not limited in the type as long as it is a material capable of initiating a curing reaction by a light surface, and for example, at least one selected from the group consisting of IRGACURE 2959 (2-hydroxy-1-[4-(2-hydroxyethoxy)phenyl]-2-methyl-1-propanone), IRGACURE 500 (1-hydroxy-cyclohexyl-phenyl-ketone and benzophenone) and IRGACURE 754 (oxy-phenyl-acetic acid 2-[2-oxo-2-phenyl-acetoxy-ethoxy]-ethyl ester and oxy-phenyl-acetic 2-[2-hydroxy-ethoxy]-ethyl ester) may be used.

The photoinitiator may be included in an amount of 0.04 parts by weight to 0.4 parts by weight based on 100 parts by weight of the solvent of the composition according to the present disclosure, and for example, may be included in an amount of 0.06 parts by weight to 0.3 parts by weight, and preferably 0.08 parts by weight to 0.15 parts by weight. When the content of the crosslinking agent is less than the above-mentioned range, the curing process of the composition according to the present invention may not be smooth or the time taken for curing may be excessively delayed, and when the content is greater than the above-mentioned range, the radical reaction rapidly proceeds, which may cause side reactions or cause unintended effects on other components of the composition according to the present disclosure, and residual components may remain excessively after curing.

The 3D printing ink composition according to the present invention may further include additives such as a surfactant, a plasticizer, a (thermal) polymerization inhibitor, a stabilizer, an antifoaming agent, a diluent and a viscosity modifier in addition to the crosslinking agent and the photoinitiator. The additives may be included in minimum amounts capable of generating the action in an economic aspect, and preferably, may be included in an amount of 0.01% by weight to 5% by weight with respect to the total weight of the ink composition.

### (5) Solvent

In one embodiment of the present disclosure, the solvent in the composition is not limited in the type as long as it is capable of readily dissolving each composition such as the fluronic copolymer, the acrylamide monomer and the alkali metal salt, however, an aqueous solvent such as water may be used.

The solvent may be included in an amount that allows each composition in the composition to be included in a preferable range depending on the purpose of the present disclosure.

### 3D Printing Molded Article

Next, a 3D printing molded article according to the present invention will be described.

The 3D printing molded article may be a photocured molded article formed by providing the 3D printing ink composition described above to a 3D printing device to form a structure, and irradiating the formed structure with a light source such as UV.

The 3D printing device capable of manufacturing the 3D printing molded article is not limited in the detailed configuration as long as it is capable of manufacturing the 3D printing molded article through the composition according to the present disclosure, and for example, the 3D printing device may include an output unit that outputs the composition, a pressure adjusting unit that adjusts a pressure of the output unit, a temperature adjusting unit that adjusts a temperature of the output unit, a sensor unit capable of sensing the conditions so as to change or select the pressure and temperature conditions depending on the type, size and property conditions of the structure to output, and a control unit that controls the pressure adjusting unit and the temperature adjusting unit.

The 3D printing ink composition according to the present invention is in a gel state at room temperature by including the alkali metal salt together with the fluronic copolymer and the acrylamide monomer, thereby having excellent rheological properties to a degree that the structure of the 3D printing before curing (pre-curing) prepared through a method such as DIW maintains a certain shape, and by performing photocuring through irradiating a UV light source or the like thereon, a molded article having high elastic modulus and excellent ultimate tensile strength may be formed.

Accordingly, the cured molded article of the 3D printing ink composition according to one embodiment of the present invention may have elastic modulus of 0.15 MPa to 1.3 MPa, specifically 0.175 MPa to 1.2 MPa, and preferably 0.208 MPa to 1.072 MPa. The 3D printing molded article having the elastic modulus satisfying the above-described range may have excellent mechanical properties.

In addition, the cured molded article of the 3D printing ink composition according to one embodiment of the present invention may have ultimate tensile strength of 0.05 MPa to 0.8 MPa, specifically 0.075 MPa to 0.6 MPa and preferably 0.087 MPa to 0.424 MPa. The ultimate tensile strength of the 3D printing molded article may be a value measured based on an ASTM D638 specimen. The 3D printing molded article having the ultimate tensile strength satisfying the above-described range may have excellent mechanical properties.

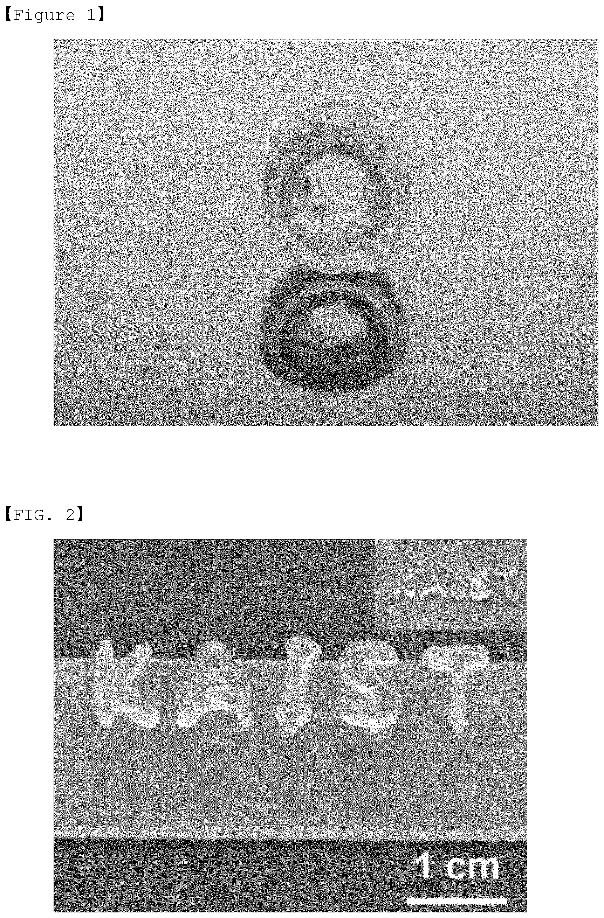

The 3D printing molded article according to the present invention may be manufactured into various shapes or types of molded articles by varying components of the 3D printing ink composition depending on the purpose. FIGS. 1 and 2 each illustrate the 3D printing molded article manufactured using the 3D printing ink composition according to the present disclosure. The 3D printing molded article according to the present invention may also be manufactured into a molded article having a multilayer with different properties by varying components of each composition. In addition, the 3D printing ink composition includes the acrylamide monomer, which is one type of biocompatible hydrogel, as a main component, and therefore, may be more suitably applied to artificial organs and the like by utilizing such properties.

Hereinafter, specific examples of the present invention will be provided. However, the examples described below are only to specifically illustrate or explain the present disclosure, and the present invention is not limited thereby. In addition, description not provided herein may be sufficiently technically inferred by those skilled in the art, and therefore, the description is not included.

### Preparation Example: Synthesis of Pluronic Copolymer

(1) In a vacuum reactor, PF127 (Pluronic F127) (100 g), a pluronic copolymer, was introduced to dichloromethane (DCM) (1,000 mL), a solvent, and dissolved therein by sufficient stirring. Triethylamine (6 mL) was additionally introduced thereto and mixed therewith.
(2) Nitrogen gas was refluxed in the reactor to create a nitrogen atmosphere, and methacryloyl chloride (4 mL) was slowly injected into the reactor. After that, the reaction was conducted for about 24 hours at room temperature while stirring the reactor.
(3) After the reaction was finished, the mixture solution was vacuum filtered to remove impurities, and then an evaporation device was installed in the reactor to remove the residual solvent and to obtain a reaction-completed product. The product was washed two or more times using a large amount of ether, and the residual ether was sufficiently evaporated to prepare a pluronic copolymer represented by Formula 2 below.

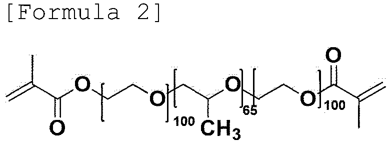

### Example and Comparative Example: Preparation of 3D Printing Ink Composition

The pluronic copolymer prepared in Preparation Example, and acrylamide were introduced to distilled water, a solvent, and sufficiently dissolved therein. After that, N,N'-methylenebisacrylamide (MBA), a crosslinking agent, and IRGACURE 2959 (2-hydroxy-1-[4-(2-hydroxyethoxy)phenyl]-2-methyl-1-propanone), a photoinitiator, were introduced thereto together, and mixed therewith. Bubbles generated during the mixing were sufficiently removed. NaCl was introduced to the mixture solution as an alkali metal salt to prepare an ink composition.

The 3D printing ink composition was prepared by employing the components of the pluronic copolymer, the acrylamide, the crosslinking agent, the photoinitiator and the alkali metal salt each in the content ratio according to Tables 1 to 3 below in the composition.

### Experimental Example 1: Measurement of Micelle Formation and Size of Pluronic Copolymer and Evaluation of Sol-Gel Tendency of 3D Printing Ink Composition

In the 3D printing ink composition according to each of Examples 1 and 2, and Comparative Example 1 to 5 of Table 1 below, the size of micelle formation of the pluronic copolymer was measured to evaluate the degree of gelation of the composition.

In this case, the micelle size of the pluronic copolymer was measured through DLS (dynamic light scattering) data of the micelles.

**[Table 1]**

| | Solvent (mL) | Acryla mide (g) | Pluronic Copolyme r (g) | NaCl (g) | MBA (g) | IRGACURE 2959 (g) | Unimer Size (nm) | Micelle Size (nm) |
|---|---|---|---|---|---|---|---|---|
| Example 1 | 4.75 | 0.25 | 0.25 | 0.1388 | 0.00015 | 0.00037 | 6.20 | 37.84 |
| Example 2 | 4.75 | 0.25 | 0.25 | 0.2776 | 0.00015 | 0.00037 | - | 24.36 |
| Comparative Example 1 | 4.75 | 0 | 0.25 | 0 | 0 | 0 | 4.85 | 50.74 |
| Comparative Example 2 | 4.75 | 0.1218 | 0.25 | 0 | 0.00007 | 0.00019 | 7.53 | 68.06 |
| Comparative Example 3 | 4.75 | 0.25 | 0.25 | 0 | 0.00015 | 0.00037 | 6.50 | 91.28 |
| Comparative Example 4 | 4.75 | 0 | 0.25 | 0.1388 | 0 | 0 | - | 32.67 |
| Comparative Example 5 | 4.75 | 0 | 0.25 | 0.2776 | 0 | 0 | - | 21.04 |

| | | | | | | | | |
|---|---|---|---|---|---|---|---|---|
| (In Table 1, 'unimer' means a pluronic copolymer that was not able to form 'micelle') (1) Referring to FIG. 5, the composition according to Comparative Example 1 includes only the pluronic copolymer, and since the pluronic copolymer itself has a tendency to be in a gel state, it may be seen that most of the pluronic copolymer in the composition exist in a micelle form. | | | | | | | | |

The composition according to Comparative Example 3 further includes acrylamide in the composition according to Comparative Example 1, and it may be seen that, by including the acrylamide, micelle formation of the pluronic copolymer is inhibited, and most of the pluronic copolymer are released into a unimer state. Accordingly, it may be seen that the acrylamide changes the tendency of the composition into a sol state.

The composition according to Comparative Example 5 further includes NaCl that is the alkali metal salt in the composition according to Comparative Example 1. It may be identified that most of the pluronic copolymer exist as micelles with almost no unimers, and through this, it may be seen that the alkali metal salt facilitates micelle formation of the pluronic copolymer in the composition, and induces aggregation of the formed micelles, thereby allowing the composition to have more gel properties.

The composition according to Example 1 further includes the acrylamide and the alkali metal salt in the composition according to Comparative Example 1, and it may be seen that, even when the acrylamide having a property of changing the tendency of the composition to a sol state is included, the alkali metal salt performs a role of aggregating micelles of the pluronic copolymer, and accordingly, micelles are smoothly formed without forming unimers of the pluronic copolymer, and the tendency of the composition is changed into a gel state.

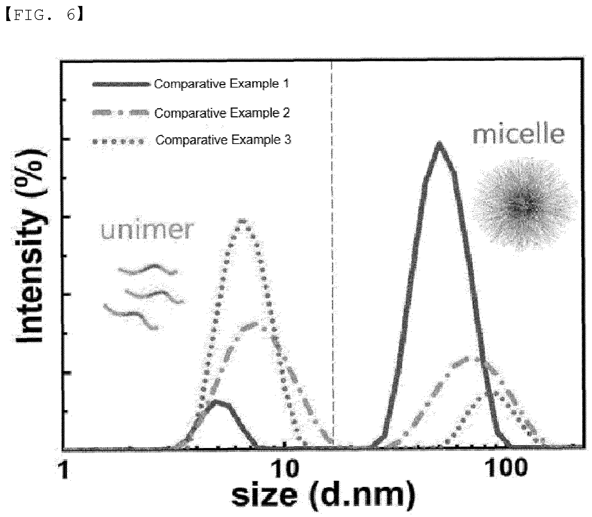

(2) Referring to FIG. 6, it may be seen that, in Comparative Examples 1 to 3, the amount of unimers increases and the micelle size increases in the pluronic copolymer as the content of the acrylamide increases in the composition, and accordingly, it may be seen that the tendency of the composition is changed into a sol state.

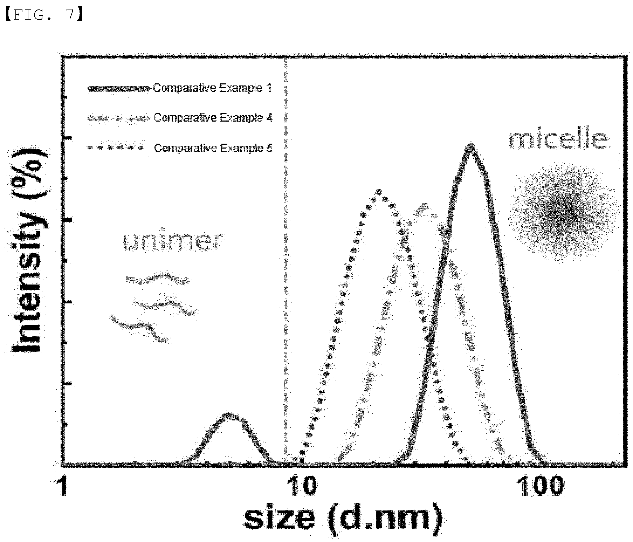

(3) Referring to FIG. 7, it may be seen that, in Comparative Examples 1, 4 and 5, the micelle size decreases in the pluronic copolymer as the content of the alkali metal salt increases in the composition, and accordingly, it may be seen that the tendency of the composition is changed into a gel state.

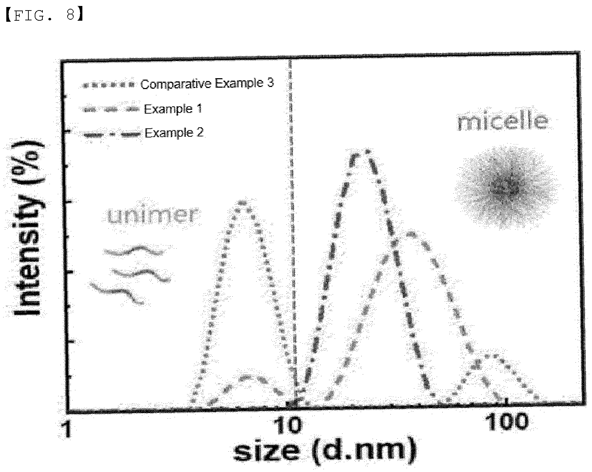

(4) Referring to FIG. 8, it may be seen that, in Examples 1 and 2, and Comparative Example 3, micelle formation is smooth and the size is reduced in the pluronic copolymer as the content of the alkali metal salt increases in the presence of the same amount of acrylamide in the composition, and accordingly, it may be seen that the tendency of the composition is changed into a gel state.

### Experimental Example 2: Measurement and Evaluation of Gelation Temperature, Rheological Properties and Biocompatibility of 3D Printing Ink Composition

(1) For the 3D printing ink composition according to each of Examples 3 to 8, and Comparative Example 6 of Table 2 below, the gelation temperature of the composition depending on a change in the content of the alkali metal salt was measured to evaluate the degree of gelation of the composition.

**[Table 2]**

| | Solvent (mL) | Acrylam ide (g) | Pluronic Copolymer (g) | NaCl (g) | MBA (g) | IRGACURE 2959 (g) | Gelation Temperat ure (°C) |
|---|---|---|---|---|---|---|---|
| Example 3 | 3.25 | 2.143 | 1.75 | 0.189 (1.0 M) | 0.001286 | 0.00325 | 47 |
| Example 4 | 3.25 | 2.143 | 1.75 | 0.2835 ( 1.5 M) | 0.001286 | 0.00325 | 42.5 |
| Example 5 | 3.25 | 2.143 | 1.75 | 0.378 (2.0 M) | 0.001286 | 0.00325 | 38 |
| Example 6 | 3.25 | 2.143 | 1.75 | 0.475 (2.5 M) | 0.001286 | 0.00325 | 31 |
| Example 7 | 3.25 | 2.143 | 1.75 | 0.567 (3.0 M) | 0.001286 | 0.00325 | 27.5 |
| Example 8 | 3.25 | 2.143 | 1.75 | 0.6615 (3.5 M) | 0.001286 | 0.00325 | 23.5 |
| Comparative Example 6 | 3.25 | 2.143 | 1.75 | 0.0 (0.0 M) | 0.001286 | 0.00325 | 57.5 |

The gelation temperature of the composition was measured through storage modulus (G') and loss modulus (G") depending on a change in the temperature of the 3D printing ink composition, which is a viscoelastic fluid, according to each of Examples 3 to 8, and Comparative Example 6.

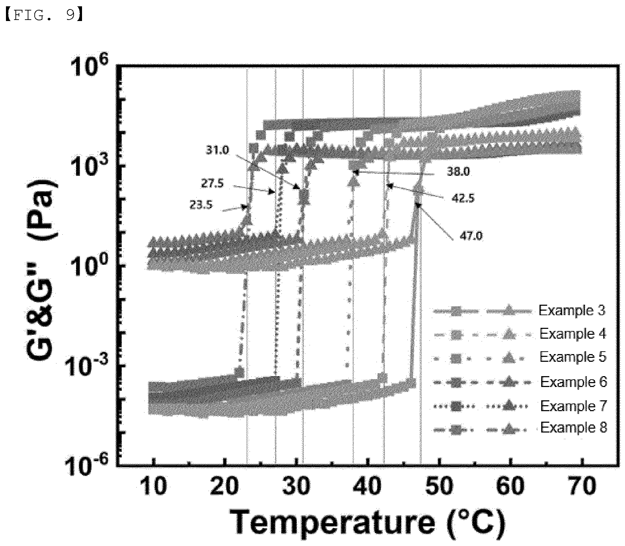

Referring to FIG. 9, it may be identified that, when the 3D printing ink composition includes the alkali metal salt, the gel tendency of the composition becomes stronger, and as the content increases within a certain range, the gelation temperature of the composition is lowered. Through this, it may be seen that, when the 3D printing ink composition according to the present invention includes a certain amount or more of the alkali metal salt, a composition having 'gel' properties even at room temperature may be formed without introducing a separate heat source by lowering the gelation temperature of the composition, and properties of an ink composition suitable for DIW-type 3D printing may be obtained, even when the composition includes the acrylamide monomer.

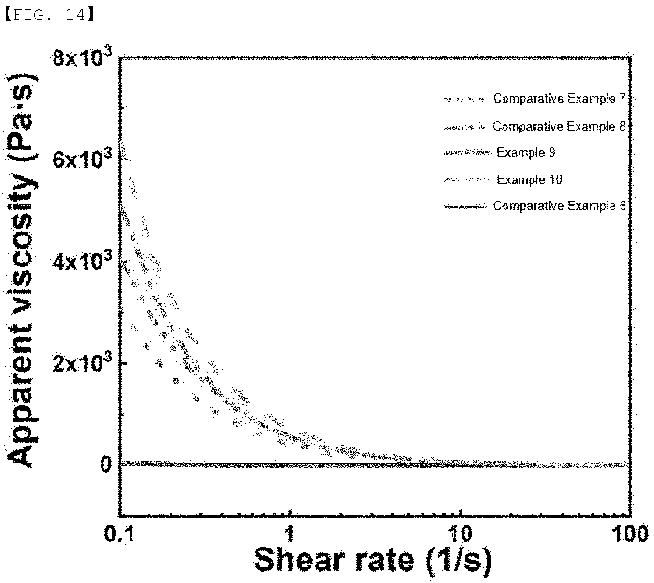

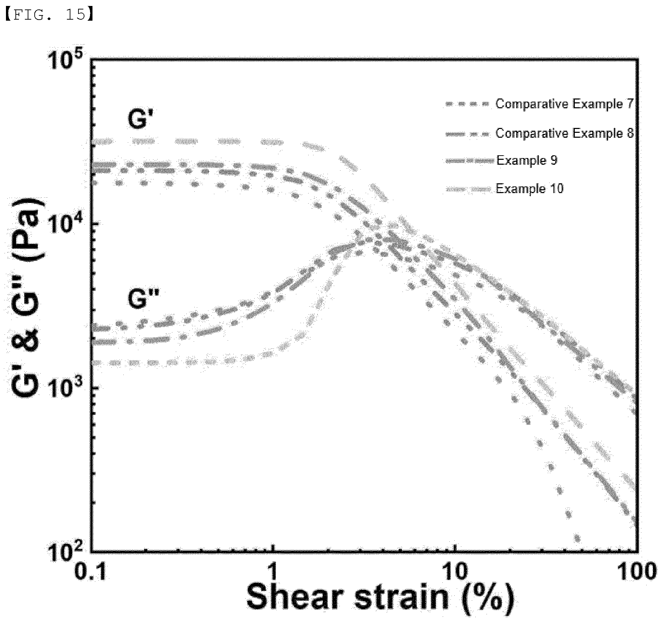

(2) For the 3D printing ink composition according to each of Examples 9 and 10, and Comparative Example 6 to 8 of Table 3 below, rheological properties of the composition depending on a change in the content of the alkali metal salt were measured to evaluate the degree of 3D printability of the composition, and the results are shown in FIGS. 14 and 15.

**[Table 3]**

| | Solvent (mL) | Acrylamide (g) | Pluronic Copolymer (g) | NaCl (g) | MBA (g) | IRGACURE 2959 (g) |
|---|---|---|---|---|---|---|
| Example 9 | 3.25 | 1.25 | 1.75 | 0.189(1.0 M) | 0.00033 | 0.00084 |
| Example 10 | 3.25 | 2.143 | 1.75 | 0.475(2.5 M) | 0.00075 | 0.0019 |
| Comparative Example 6 | 3.25 | 2.143 | 1.75 | 0.0(0.0 M) | 0.001286 | 0.00325 |
| Comparative Example 7 | 3.25 | 0.0 | 1.75 | 0.0 | 0.0 | 0.00042 |
| Comparative Example 8 | 3.25 | 0.556 | 1.75 | 0.0 | 0.001286 | 0.00325 |

Referring to FIG. 14, it may be identified that the composition according to Comparative Example 6 not including the alkali metal salt has low composition viscosity like water compared to the compositions of Example 9, Example 10, Comparative Example 6 and Comparative Example 7 further including the alkali metal salt in the composition, and the composition according to Comparative Example 6 has viscosity not suitable for 3D printing.

In addition, it may be identified that, in the composition according to each of Example 9, Example 10, Comparative Example 6 and Comparative Example 7 further including the alkali metal salt in the composition, viscosity decreases as the shear rate increases as shown in FIG. 14, and storage modulus (G') and loss modulus(G") are reversed based on a certain value as the shear strain increases as shown in FIG. 15, indicating that a shear thinning phenomenon, an essential property for extrusion-based 3D printing such as the DIW method, occurs.

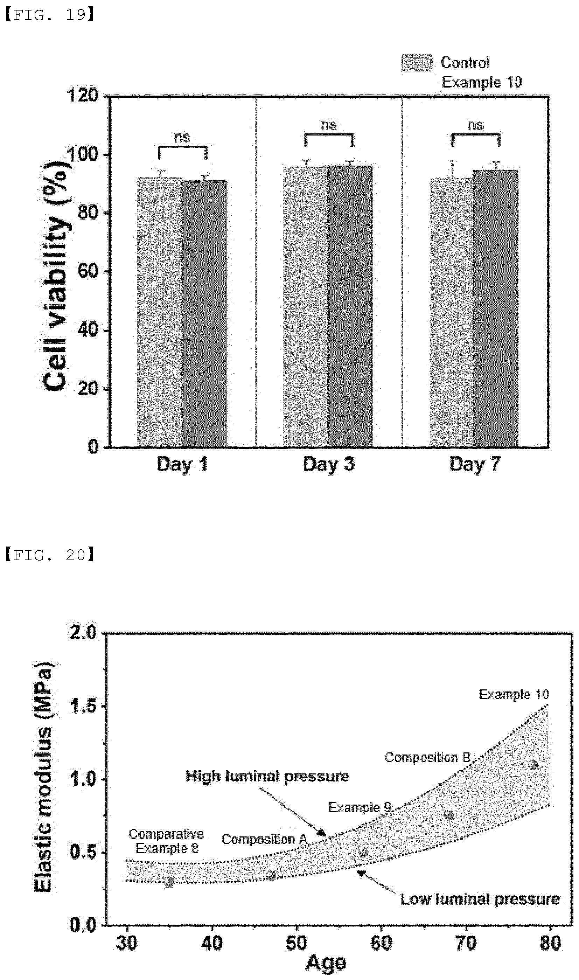

(3) Biocompatibility (cell viability) of the 3D printing ink composition according to Example 10 of Table 3 was measured, and the results are shown in FIGS. 18 and 19.

In order to measure biocompatibility (cell viability) of the 3D printing ink composition, NIH-3T3 fibroblast cells (0.5×10⁵ cells/mL) cultured for 72 hours at 37°C in a DMEM (Dulbecco's modified eagle medium) to which 10% BCS (bovine calf serum) and 1% PS (penicillin-streptomycin) were added was employed as a control group, and 5×5×1 mm³ of the 3D printing ink composition according to Example 10 was introduced to the control group in a medium to be employed as an experimental group, and cell viability in the media was observed for 7 days using a live/dead kit (Invitrogen, L3224).

Referring to FIGS. 18 and 19, it was identified that almost no dead cells were observed in the medium to which the 3D printing ink composition according to Example 10 was introduced when observing cell viability for 7 days, which may identify that the 3D printing ink composition according to the present invention has excellent biocompatibility (cell viability). FIG. 18 illustrates fluorescence images of the media of the control group and the experimental group using a confocal laser scanning microscope (CLSM) of 10x magnification.

### Experimental Example 3: Measurement and Evaluation of Elastic Modulus, Tensile Strength, Electric Conductivity and Printing Controllability of 3D Printing Molded Article

(1) Using each of the 3D printing ink compositions according to Examples 9 and 10, Comparative Examples 7 and 8, and Compositions A and B of Table 4 below, a structure was formed by performing DIW-type 3D printing, and the structure was irradiated with a UV light source and photocured to manufacture a 3D printing molded article (artificial vessel). Elastic modulus and ultimate tensile strength were measured for each of the molded articles to evaluate properties of the molded article.

**[Table 4]**

| | Solvent (mL) | Acryl amide (g) | Pluronic Copolyme r (g) | NaCl (g) | MBA (g) | IRGACURE 2959 (g) | Elastic Modulus (MPa) | Ultimate Tensile Strength (MPa) |
|---|---|---|---|---|---|---|---|---|
| Example 9 | 3.25 | 1.25 | 1.75 | 0.189 | 0.00033 | 0.00084 | 0.294 | 0.160 |
| Example 10 | 3.25 | 2.143 | 1.75 | 0.475 | 0.00075 | 0.0019 | 0.584 | 0.311 |
| Comparative Example 7 | 3.25 | 0.0 | 1.75 | 0.0 | 0.0 | 0.00042 | 1.072 | 0.424 |
| Comparative Example 8 | 3.25 | 0.556 | 1.75 | 0.0 | 0.001286 | 0.00325 | 0.208 | 0.087 |
| Composition A | 3.25 | 0.882 | 1.75 | 0.095 | 0.00023 | 0.00133 | 0.339 | - |
| Composition B | 3.25 | 1.667 | 1.75 | 0.617 | 0.00044 | 0.00252 | 0.752 | - |

The elastic modulus (Young's modulus) was measured using a method of, while elongating each sample of Examples and Comparative Examples using a tensile tester (manufactured by Mark company) at a speed of 200 mm/min until it is cut, plotting stress-strain correlation as a graph and then calculating the initial slope value in the elastic region in the corresponding graph.

The ultimate tensile strength was measured using a method of, while elongating each sample of Examples and Comparative Examples using a tensile tester (manufactured by Mark company) at a speed of 200 mm/min until it is cut, plotting stress-strain correlation as a graph and then measuring the maximum stress value in the corresponding graph.

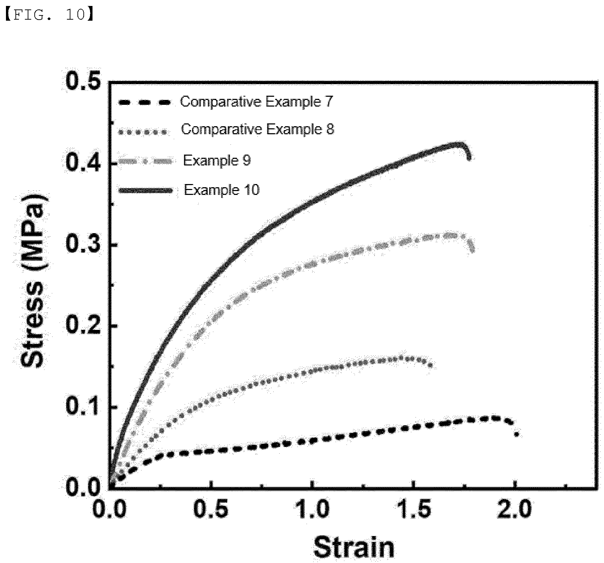

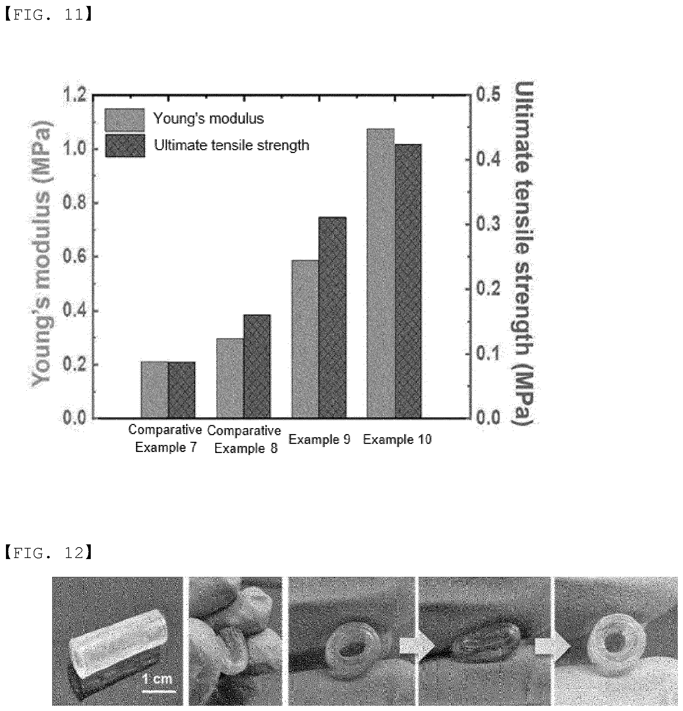

Referring to FIGS. 10 and 11, the tendency of increasing elastic modulus and ultimate tensile strength may be identified as the composition includes acrylamide, and it may be seen that elastic modulus and ultimate tensile strength of the 3D printing molded article become more superior when the composition further includes the alkali metal salt in addition to the acrylamide.

In addition, referring to FIG. 20, a wide range of mechanical properties may be covered through controlling the composition included in each of the compositions. Particularly, since the ranges of the properties and blood vessels in the human body are similar, elastic modulus increases as the elasticity of blood vessels is weakened with age, and it may be seen that the molded article using the 3D printing ink composition according to the present invention may diversely simulate such age-related tendencies, enabling more detailed medical simulations.

(2) A structure was formed by performing DIW-type 3D printing using the composition according to Example 10, and the structure was irradiated with a UV light source and photocured to manufacture a band-shaped 3D printing molded article, and the ultimate tensile strength was visually evaluated.

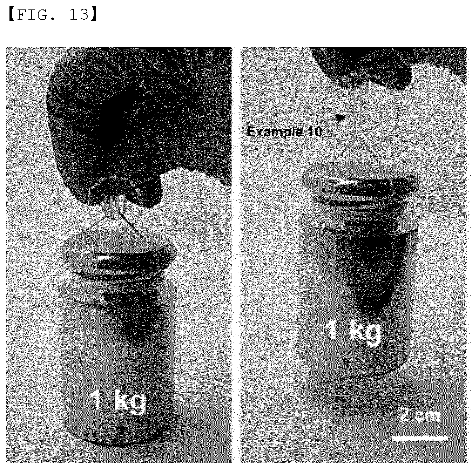

Referring to FIG. 13, it may be identified that the 3D printing molded article formed using the composition of Example 10 is not broken even in an environment in which external force of lifting a 1 kg weight in a vertical direction is applied.

(3) A 3D printing molded article was manufactured in the same manner as in (1) using each of the 3D printing ink compositions according to Example 8 and Comparative Example 6 of Experimental Example 2, and electric conductivity of the 3D printing molded article was measured at 25°C using an electric conductivity meter (Hioki, L2000 4-terminal probe).

Referring to Table 5 below, it may be identified that the 3D printing molded article formed from the 3D printing ink composition as in Example 8 had significantly improved electric conductivity compared to the 3D printing molded article formed from the 3D printing ink composition according to Comparative Example 6 by further including the alkali metal salt in the composition.

**[Table 5]**

| | Solvent (mL) | Acryl amide (g) | Pluronic Copolyme r (g) | NaCl (g) | MBA (g) | IRGACURE 2959 (9) | Electric Conductivi ty (mS/m) |
|---|---|---|---|---|---|---|---|
| Example 8 | 3.25 | 2.143 | 1.75 | 0.6615 (3.5 M) | 0.001286 | 0.00325 | 1,345 |
| Comparative Example 6 | 3.25 | 2.143 | 1.75 | 0.0 (0.0 M) | 0.001286 | 0.00325 | 201.3 |

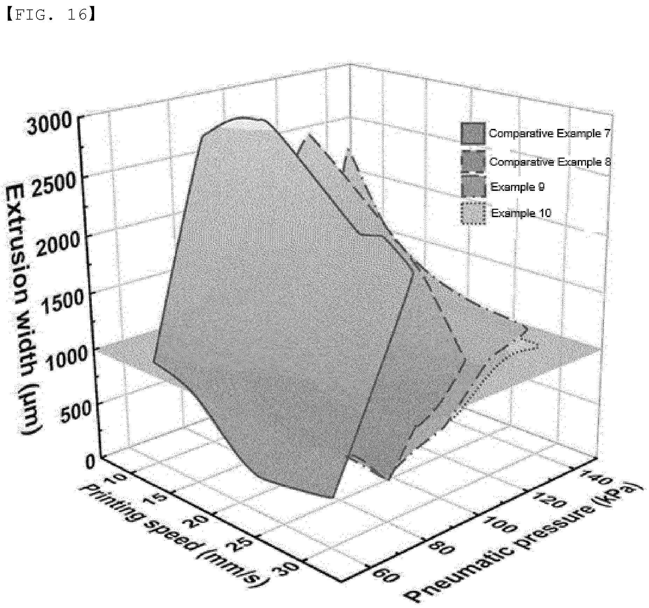

(4) DIW-type 3D printing was performed using each of the compositions according to Examples 9 and 10, and Comparative Examples 7 and 8 of Experimental Example 2, and extrusion pressure and printing speed of the 3D printing were adjusted so as to form a structure having a width of 1 mm. The results are shown in FIG. 16.

Referring to FIG. 16, it may be identified that, although the compositions according to Examples 9 and 10, and Comparative Examples 7 and 8 have different rheological properties by having different amounts of the components such as the pluronic copolymer, the acrylamide and the alkali metal salt included in each of the compositions, multi-nozzle printing is possible in performing DIW-type 3D printing by adjusting variables of the nozzle unit to have the same width.

(5) Using the 3D printing ink composition of each of Examples 9 and 10, and Compositions A and B of Table 4, a structure was formed by performing DIW-type 3D printing, and the structure was irradiated with a UV light source and photocured to manufacture an artificial vessel that is a 3D printing molded article, and the elastic modulus was measured. The elastic modulus was measured in the same manner as in (1).

Hereinbefore, preferred embodiments of the present invention have been described in detail, however, the scope of a right of the present invention is not limited thereto, and various modifications and improvements made by those skilled in the art using the basic concept of the present invention defined in the claims also fall within the scope of a right of the present disclosure.

## Claims

1. A 3D printing ink composition, comprising:
a pluronic copolymer;
an acrylamide monomer;
an alkali metal salt; and
a solvent.

2. The composition of claim 1, wherein the pluronic copolymer is represented by Formula 1 below: 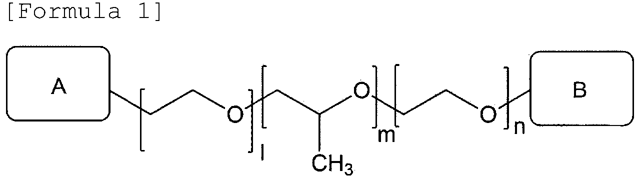 wherein,
A and B are terminal groups capable of forming a bond with the acrylamide monomer; and
l is an integer of 95 to 100, m is an integer of 62 to 69, and n is an integer of 95 to 100.

3. The composition of claim 2, wherein A of Formula 1 is represented by Structural Formula A-1 or Structural Formula A-2 below; and
B of Formula 1 is represented by Structural Formula B-1 or Structural Formula B-2 below: 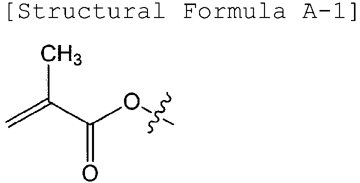 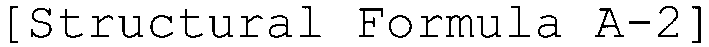 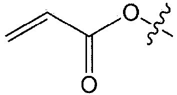 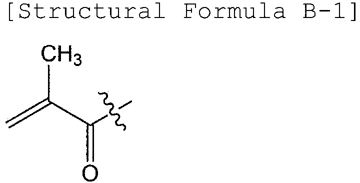 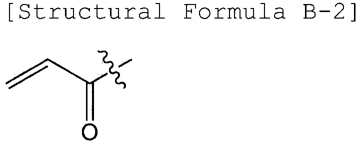
in Structural Formula A-1 to Structural Formula B-2, 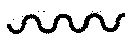 represents a site linked to Formula 1.

4. The composition of claim 1, wherein the acrylamide monomer is at least one selected from the group consisting of acrylamide, N-isopropylacrylamide (NIPAM), N-tert-butylacrylamide, N,N-diethylacrylamide, N-ethylacrylamide, N-n-propylacrylamide, N,N-ethylmethylacrylamide, N-isopropylmethacrylamide, N-hydroxyethylacrylamide, N-(isobutoxymethyl)acrylamide, N-tert-butyl methacrylamide, N,N-diethyl methacrylamide and N-ethyl methacrylamide.

5. The composition of claim 1, wherein the alkali metal salt includes a combination of at least one anion selected from the group consisting of OH⁻, SO₄²⁻, CO₃²⁻, BrO₃⁻, Cl⁻, OAc⁻, IO₃⁻, Br⁻, I⁻ and NO₃⁻, and at least one cation selected from the group consisting of Na⁺, K⁺, Li⁺, Ba²⁺, Rb⁺, Ca²⁺, Ni²⁺, Co²⁺, Mg²⁺, Fe²⁺, Zn²⁺, Cs⁺, Mn²⁺, Al³⁺, NH⁴⁺ and H⁺.

6. The composition of claim 1, further comprising:
a crosslinking agent; and
a photoinitiator.

7. The composition of claim 6, wherein the crosslinking agent is at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin and ethylene carbonate.

8. The composition of claim 6, wherein the photoinitiator is at least one selected from the group consisting of IRGACURE 2959 (2-hydroxy-1-[4-(2-hydroxyethoxy)phenyl]-2-methyl-1-propanone), IRGACURE 500 (1-hydroxy-cyclohexyl-phenyl-ketone and benzophenone) and IRGACURE 754 (oxy-phenyl-acetic acid 2-[2-oxo-2-phenyl-acetoxy-ethoxy]-ethyl ester and oxy-phenyl-acetic 2-[2-hydroxy-ethoxy]-ethyl ester).

9. The composition of claim 1, comprising the pluronic copolymer in an amount of 15 parts by weight to 80 parts by weight based on 100 parts by weight of the solvent.

10. The composition of claim 1, comprising the acrylamide monomer in an amount of 5 parts by weight to 100 parts by weight based on 100 parts by weight of the solvent.

11. The composition of claim 1, comprising the alkali metal salt in an amount of exceeding 0 parts by weight and 25 parts by weight or less based on 100 parts by weight of the solvent.

12. The composition of claim 6, comprising the crosslinking agent in an amount of 0.0037 parts by weight to 0.074 parts by weight based on 100 parts by weight of the solvent.

13. The composition of claim 6, comprising the photoinitiator in an amount of 0.04 parts by weight to 0.4 parts by weight based on 100 parts by weight of the solvent.

14. The composition of claim 1, wherein the acrylamide monomer and the alkali metal salt in the composition have a weight ratio of 2:1 to 20:1.

15. The composition of claim 1, wherein a gelation temperature of the composition is 5°C to 60°C.

16. A 3D printing molded article formed by curing the composition of any one of claims 1 to 15.

17. The molded article of claim 16, wherein an elastic modulus of the 3D printing molded article is 0.15 MPa to 1.3 MPa.

18. The molded article of claim 16, wherein an ultimate tensile strength of the 3D printing molded article is 0.05 MPa to 0.8 MPa.
